# EUROPEAN PATENT SPECIFICATION

(11) **EP 0 387 451 B1**
(45) Date of publication and mention of the grant of the patent: **08.03.1995**
(21) Application number: 89313314.0
(22) Date of filing: 19.12.1989
(51) Int. Cl.: A61B 17/22, A61B 17/32, A61M 29/02

(54) **Atherectomy system**
Atherektomiesystem
Système d'athérectomie

(30) Priority: 13.03.1989 US 322497
(43) Date of publication of application: 19.09.1990
(73) Proprietor: Shiber, Samuel, Atkinson, NH 03811 (US)
(72) Inventor: Shiber, Samuel, Atkinson, NH 03811 (US)
(74) Representative: Woodward, John Calvin

(56) References cited:
- EP-A- 0 163 502
- EP-A- 0 238 217
- US-A- 4 627 436
- US-A- 4 732 154
- US-A- 4 772 258
- US-A- 4 842 579

## Description

This invention relates to an atherectomy system for coring, ingesting and removing an obstruction from within a patient's blood vessel.

With age a large percentage of the population develops atherosclerotic arterial obstructions resulting in a diminished blood circulation. The disturbance to blood flow that these obstructions cause may induce blood clots which further diminish or block the blood flow. When this process occurs in the coronary arteries it is referred to as a heart attack. Presently such obstructions are circumvented by surgically grafting a bypass or they are treated by a catheter equipped with a balloon which is inserted through the arterial system, over a flexible guide-wire, into the obstruction and then inflated to expand the obstruction's lumen (angioplasty). Some of the problems with angioplasty are that it injures the arterial wall, it creates a rough lumen and in a substantial number of the cases it is ineffective. Further, angioplasty does not remove the obstruction material out of the arterial system, therefore, in the case of a heart attack, immediate angioplasty carries the risk of dislodging the blood clot and allowing it to move down stream creating additional blockages.

According to the invention, there is provided an atherectomy system for coring, ingesting and removing an obstruction from within a patient's blood vessel, characterised by a flexible guide-wire insertable into the vessel, a flexible rotary catheter rotatably disposed and insertable into the vessel over said flexible guide-wire, having at its distal end a rotary coring means for making a circular cut in an obstruction located in front of it, and coupling means at its proximal end, a continuous passage for ingesting the cored obstruction material, defined between said flexible rotary catheter and said flexible guide-wire, where the relative motion between said flexible rotary catheter and said flexible guide-wire assists in moving said ingested obstruction material proximally in said continuous passage, and a sleeve in which said flexible rotary catheter is slidably and rotatably disposed, having a window near its open distal end.

The preferred atherectomy catheter of the invention is insertable and advancable in an artery over a flexible guide-wire. The flexible rotary catheter is equipped with a coring means at its distal end to core and extract obstruction material and create a smooth lumen without cracking the arterial wall. The catheter is slidable in a sleeve having a window near its distal open end. Obstruction material can enter the sleeve through the window and the open distal end, to be cored and ingested by the flexible rotary catheter. The sleeve is slidable over the catheter, or over a standard dilator, for insertion thereof into an artery and further into an obstruction. This facilitates treatment of eccentric lesions and the cleaning of larger arteries through a smaller puncture at the point of insertion of the system into the vessel.

Preferred embodiments of the invention will now be described, by way of example only, with reference to the accompanying drawings, in which :
Figure 1 schematically shows a general view of an atherectomy system inserted at the groin area, through a patient's arterial system into his obstructed coronary artery,
Figure 2 shows a cross sectioned view of the atherectomy system. The middle portion of the system is represented with a phantom line, due to space limitations on the drawing sheet.
Figure 3 shows a bottom view of a distal portion of an embodiment of the system.
Figure 4 shows a side view of a distal portion of the embodiment, in an artery, prior to pressurizing an inflatable chamber and prior to expanding a barrier means at the distal end of the flexible guide-wire.
Figure 5 shows a cross sectional view along line 5-5 marked on Figure 4 of the embodiment, in an artery, prior to pressurizing the inflatable chamber.

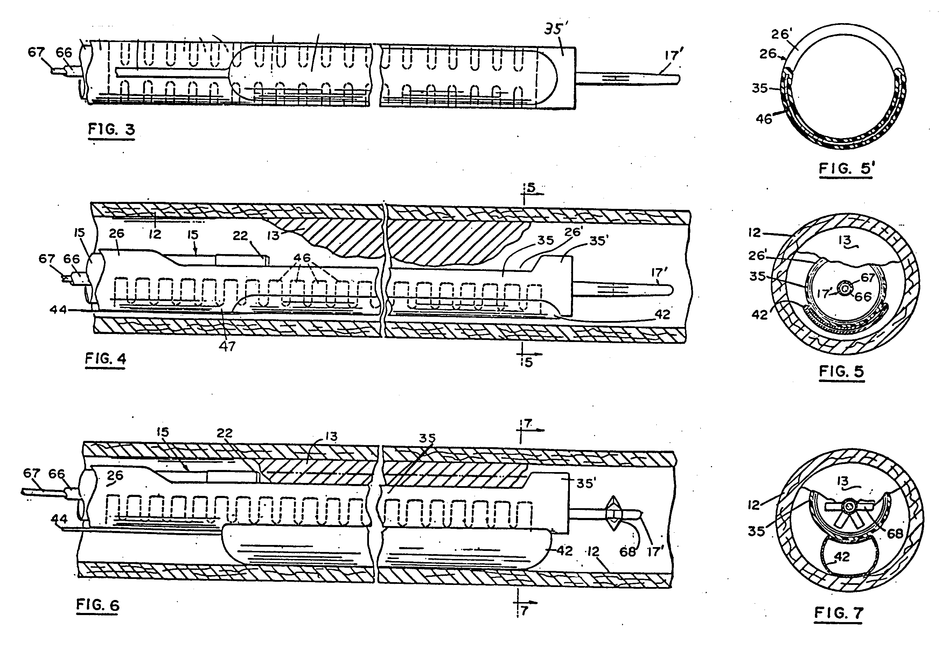

Figure 5′ shows a cross sectional view along line 5-5 marked on Figure 4 of the window region of a sleeve of the embodiment, on an enlarged scale, showing the reinforcing rib.
Figure 6 shows a side view of a distal portion of the embodiment, in an artery, with the inflatable chamber pressurized through the inflatable tube. Obstruction material is accepted into a sleeve through a window in order to be cored and removed along a phantom line. The distal barrier means are expanded.
Figure 7 shows a cross sectional view along line 7-7 marked on Figure 6 of the embodiment, in an artery, with the inflatable chamber pressurized and with the distal barrier means expanded.

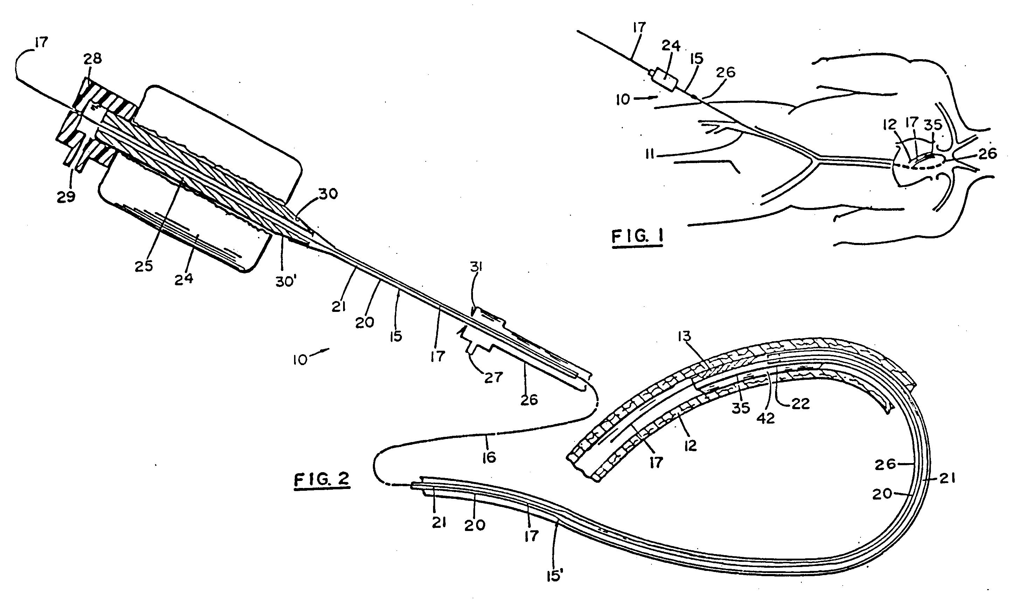

Figure 1 schematically shows a general view of an atherectomy system with a sleeve 10, introduced percutaneously (through the skin) into a patient's artery 11 at the groin area. The system's distal end is inserted through the arterial system to reach a work site in a coronary artery 12 while its proximal end remains outside the patient's body.

As shown in Figure 2 the atherectomy system comprises several elongated parts in a nested relationship, and their ends shall be referred to as "distal" meaning the end which goes into the vessel and "proximal" meaning the other end, thus, "distal direction" or "distally" shall indicate a general direction from the proximal end to the distal end, and "proximal direction" or "proximally" shall refer to an opposite direction.

As further shown in Figure 2, the distal portion of the system 10 is placed in the diseased coronary artery 12 (same numbers are used to indicate similar items throughout the Figures) containing an eccentric atherosclerotic obstruction 13. The system's mid portion is represented by a phantom line 16 because of space limitation of the drawing sheet.

The system comprises:
A flexible guide wire 17 insertable through the human vascular system.
A flexible rotary catheter 15, rotatable and slidable over the guide wire, having a wall 20 defining a longitudinal channel 21. Coring means in the form of a tubular blade 22 is mounted at the distal end of the flexible rotary catheter. The tubular blade defines a through-hole which forms with the channel 21 a continuous passage for accepting the obstruction material ingested into the through-hole. A variety of rotary coring means have been shown in my incorporated by reference applications: Tubular blades, single tooth blades, blades which are essentially the distal end of the flexible rotary catheter and radiation emitting devices. Some of these rotary coring means are incorporated into, or are part of, the distal end of the flexible rotary catheter and have no internal wall of their own, in which case the continuous passage consists of the channel 21.

Frictional losses which develop between the flexible rotary catheter and a sleeve 26 strain the flexible rotary catheter and the motor. These frictional losses are more pronounced with an obese patient where the system is introduced into the femoral artery at the groin and has to assume a sharp bend at the point of entry due to patient's protruding abdomen. To reduce these frictional losses the diameter of the proximal portion of the flexible rotary catheter is decreased at a point marked 15′ on Figure 2. Such a diametrical decrease does not interfere with the ingestion of obstruction material as long as a sufficient distal portion of a full diameter flexible rotary catheter is left to accept the material. For example, a flexible rotary catheter for removing an obstruction from peripheral arteries having a total length of 50cm can be manufactured with a 35cm proximal portion of 2mm diameter and a 15cm distal portion of 3mm diameter. However, if the obstruction is long the location of the diametrical transition point 15′ can be moved proximally to increase the catheter's material ingestion capacity.

A motor 24 has a hollow shaft 25 with a tapered distal end 30′ which engages a coupling means in the form of a matching tapered seat 30 formed at the proximal end of the flexible rotary catheter.

The sleeve 26 has a window region 35 with a biasing means near its open distal end. The sleeve defines in the vessel a trajectory for the coring means to move along and it can also serve as an introducer of the system into the artery. Alternatively, the sleeve can be introduced through a separate introducing sheath. The sleeve has a port 27 through which it accepts contrast or irrigating, lubricating and/or radio opaque contrast fluids and delivers them through its distal end to the work site. A seal 31 prevents the fluid from escaping through the sleeve's proximal end.

A rotary joint 28 has a port 29 which is connected through the hollow shaft 25 to the continuous passage and can be used for introducing a negative pressure in the continuous passage to assist in sucking and drawing the cored obstruction material into it. The suction and a mechanical action resulting from the relative motion between the rotating inner wall of the continuous passage and the flexible guide wire effectively ingests the cored material into the continous passage for subsequent removal thereof out of the patient's body. In an early stage of the procedure, prior to ingesting obstruction material, the fluid route connecting the port 29 and the distal end of the flexible rotary catheter can be used as an alternative route for delivering fluids to the work site. The flexible guide wire slidably passes through a close fitting hole formed at the proximal end of the rotary joint.

Figures 3 to 7 show the distal end of an atherectomy system with a sleeve for removing an obstruction from within the patient's vessel, artery 12, comprising:
the flexible guide wire 17′,
the flexible rotary catheter 15 with coring means in the form of the tubular blade 22 at its distal end, for coring and ingesting obstruction material, insertable into the vessel over the flexible guide wire.

The sleeve 26 in which the flexible rotary catheter is rotatably and slidably disposed and which in turn is slidable over the flexible rotary catheter, has an open proximal end, an open distal end 35′ and a window 26′ located near the distal end. The window is intended to be placed in the arterial lumen so that the obstruction material is urged to enter it through the window and then the material is cored and ingested by the advancing flexible rotary catheter along the phantom line which is marked on Figure 6. The window region has cross section in the shape of an incomplete circle, shown in Figures 5, 5′ and 7, which positively contains the flexible rotary catheter by surrounding more than half of its periphery. The window region's cross section is stabilized by reinforcing means in the form of semicircular rib like elements 46 (note Figure 5′). The elements 46 are tied together by a spine like element 47 which substantially contributes to the torque transmission ability of the window region while allowing it to bend. Therefore, elements 46 and 47, which can be made from flat stainless steel sheet material, stabilize the cross section of the window region to maintain a proper relationship between the window region's cross section and the tubular blade but they allow the window region to bend and conform to the arterial curvature and they enable the user to rotate the window in the artery to position it toward the obstruction material.

The window region has a selectively actuable biasing means in the form of an inflatable chamber 42 for biasing the window towards the obstruction material so that the material is urged to enter the sleeve through the window, preparatory to the flexible rotary catheter advancing distally and rotating in the sleeve, over the window, coring and ingesting this material. Then the window can be rotated a part of a turn, the flexible rotary catheter can be proximally retracted in the sleeve past the window, which is then re-biased toward the remaining obstruction material and additional pass or passes can be made as needed. Thus, eccentric obstructions and large diameter arteries can be de-bulked and re-canalizing through a smaller puncture wound in the vessel's wall.

An inflatable tube 44, for inflating the chamber 42, is formed integral with the chamber. When the chamber is inflated the tube is also slightly inflated, as shown in Figure 6, however, prior to inflation the the tube remains flat and negative pressure can be applied to the tube to further flatten it as shown in Figure 4, minimizing the tube's contribution to the size of the puncture wound that will be needed to introduce the structure into the patient's body. Alternatively, the inflation tube can be defined in the wall of the sleeve particularly if the sleeve is an extrusion, in which case the tube can be co-extruded with the main lumen of the sleeve. The inflation lumen is connected at its distal end to the chamber and at its proximal end it is connectable to inflation means such as a syringe.

The flexible guide wire 17′ has a distal barrier means in the form of thin jacket 66 over a core wire 67. The jacket and core wire are bonded at their distal tip, and by pulling the core wire and sliding it proximally relative to the jacket, the arms 68, which are formed by multiple slits in the sleeve, are extended radially. Barrier means as well as additional suitable flexible guide wire designs are discussed in my above incorporated patent applications.

The present invention provides a process for removing an obstruction from a blood vessel comprising the following steps:
Inserting into a vessel and into an obstruction a flexible guide wire.

Inserting into the vessel, over the flexible guide wire a dilator.

Inserting into the vessel over the dilator an introducer.

Withdrawing the dilator out of the introducer. As previously explained, the sleeve can also be the introducer, alternatively, the sleeve can be inserted into the vessel through the introducer.

Distally advancing over the flexible guide wire a flexible rotary catheter through the obstruction, either by coring a passage through the obstruction, or if the original lumen of the obstruction is large enough, by simply passing the catheter through it.

Inserting an open distal end of a sleeve and a window into the obstruction. The insertion of the sleeve can be done over the flexible rotary catheter, using it as a guide for safely advancing the sleeve. The clearance between the sleeve's opening at its distal end to the flexible rotary catheter is small, therefore, when the sleeve is advanced over the flexible rotary catheter the probability of its taking a big bite of arterial wall is minimized.

Retracting the coring means past the window, biasing the window against the obstruction material to cause the later to enter the sleeve through the window and then advancing and rotating the flexible rotary catheter, coring and ingesting the obstruction material that has entered the sleeve.

If the lumen through the obstruction is to be further increased by removal of additional obstruction material the above step can be repeated as needed, and prior to such repetitions the window can be rotated and moved along the artery so that it faces different areas of the obstruction.

The amount of biasing can be regulated by the inflation pressure of a chamber attached to the bottom of the window region.

Negative pressure applied to the proximal end of the continous passage can be used to cooperate with a mechanical action in enabling the cored material into the continous passage.

It should be noted that the atherectomy system can be manufactured in different diameters and lengths depending on the size and site of artery that it is intended for and on whether the system is to be used percutaneously (that is, through the skin) or intra-operatively (that is when the vessel is surgically exposed for inserting the system into a vessel). It can also be noted from the present application and the incorporated applications that components of the atherectomy system can be made in several ways: the flexible rotary catheter can be made from plastic or metal and either version can be equipped with an internal helical step. The coring means can be a tubular-blade, a heated tubular blade or a radiation emitting means such as an optical fibre located at a wall of the flexible rotary catheter carrying laser energy, or other means which core and ingest an obstruction disposed in front of it. By combining a sleeve having a certain biasing means, a certain flexible rotary catheter and a certain flexible guide wire a variety of species can be made to match the system's characteristics with the specific disease characteristics of an individual patient. Similarly, various of the steps described above can be altered, deleted or additional steps can be added to suit an individual case.

This is helpful, since the clinical characteristics of arterial atherosclerotic obstructions vary in geometry, hardness, and accessibility from one patient to another.

## Claims

1. An atherectomy system for removing an obstruction from within a patient's vessel, comprising:
a flexible guidewire (17) insertable into the patient's vessel;
a flexible rotary catheter (15) for cutting and ingesting obstruction material insertable into said vessel over the flexible guidewire, the flexible rotary catheter (15) having at its distal end a rotary coring means (22) for making a circular cut in an obstruction located in front of it and coupling means (30) at its proximal end for connection it to a power source (24), a continuous passage defined between the flexible rotary catheter (15) and the flexible guidewire (17), the flexible rotary catheter (15) being slidably and rotatable disposed in a sleeve (26) with an open distal end, characterised in that the sleeve (26) includes a window (26') near said open distal end to receive the obstruction material, said catheter being arranged to be distally movable past the window (26') to cut and ingest obstruction material in the sleeve (26).

2. An atherectomy system according to claim 1, characterised in that the sleeve (26) has a cross section which positively constrains the flexible catheter (15) within the sleeve in the region (35) of said window (26').

3. An atherectomy system according to claim 1 or claim 2 characterised in that the sleeve (26) has selectively actuable biasing means (42) provided thereon for biasing the window (26') against said obstruction.

4. An atherectomy system according to claim 3 characterised in that the biasing means comprise an inflatable chamber (42) attached to the sleeve (26) opposite the window (26').

5. An atherectomy system according to any of the preceding claims characterised by means for applying suction to a proximal end of the flexible rotary catheter (15) to pull proximally the cored obstruction material in said catheter.

6. An atherectomy system according to any preceding claim characterised in that the rotary coring means is a tubular blade (22).

7. An atherectomy system according to any preceding claim characterised in that the coring means (22) is a radiation emitting device.

8. An atherectomy system according to any preceding claim characterised in that a proximal portion of the flexible rotary catheter (15) has a decreased diameter (15') relative to the diameter in the distal section thereof.

9. An atherectomy system according to any preceding claim characterised in that the flexible guidewire (17) has radially extending distal barrier means (68) to counter distal movement of obstruction material.

10. An atherectomy system according to claim 9 characterised in that the distal barrier means (68) is elastically contractable.

11. An atherectomy system according to claim 9 or claim 10 characterised in that the distal barrier means (68) are selectively expandable.

12. An atherectomy system according to any preceding claim characterised in that means (27) are connected to the sleeve (26) for introducing fluids into the patient's vessel.

## Patentansprüche

1. Atherektomiesystem zum Entfernen einer Obstruktion aus dem Innern eines Blutgefäßes eines Patienten, umfassend
einen in das Blutgefäß des Patienten einführbaren flexiblen Führungsdraht (17),
einen über den flexiblen Führungsdraht in das Blutgefäß einführbaren flexiblen, rotierenden Katheter (15) zum Ausschneiden und Aufnehmen von Obstruktionsmaterial, wobei der flexible rotierende Katheter (15) an seinem distalen Ende ein rotierendes Ausschneidemittel (22) zum Anbringen eines kreisförmigen Schnitts in eine vor ihm liegende Obstruktion und an seinem proximalen Ende Kupplungsmittel (30) zu seinem Anschluß an einen Antrieb (24) aufweist,
einen zwischen dem flexiblen rotierenden Katheter (15) und dem flexiblen Führungsdraht (17) gebildeten ununterbrochenen Durchgang, wobei der flexible rotierende Katheter (15) verschieblich und drehbar in einer Hülse (26) mit einem offenen distalen Ende aufgenommen ist, dadurch gekennzeichnet, daß die Hülse (26) nahe ihrem offenen distalen Ende ein Fenster (26') zur Aufnahme des Obstruktionsmaterials aufweist und der Katheter so angeordnet ist, daß er distal hinter das Fenster (26') bewegbar ist, um Obstruktionsmaterial auszuschneiden und in die Hülse (26) aufzunehmen.

2. Atherektomiesystem nach Anspruch 1, dadurch gekennzeichnet, daß die Hülse (26) einen Querschnitt aufweist, der den flexiblen Katheter (15) in den Bereich (35) des Fensters (26') zwingt.

3. Atherektomiesystem nach Anspruch 1 oder 2, dadurch gekennzeichnet, daß die Hülse (26) auf ihr angeordnete wahlweise einstellbare Vorspannmittel (42) zur Vorspannung des Fensters (26') gegen die Obstruktion aufweist.

4. Atherektomiesystem nach Anspruch 3, dadurch gekennzeichnet, daß die Vorspannmittel eine an der Hülse (26) dem Fenster (26') gegenüberliegend angebrachte aufblasbare Kammer (42) beinhalten.

5. Atherektomiesystem nach irgendeinem der vorhergehenden Ansprüche, gekennzeichnet durch Mittel zur Ausübung von Unterdruck auf ein proximales Ende des flexiblen rotierenden Katheters (15), um proximal das ausgeschnittene Obstruktionsmaterial in den Katheter zu ziehen.

6. Atherektomiesystem nach irgendeinem der vorhergehenden Ansprüche, dadurch gekennzeichnet, daß das Ausschneidemittel eine rohrförmige Klinge (22) ist.

7. Atherektomiesystem nach irgendeinem der vorhergehenden Ansprüche, dadurch gekennzeichnet, daß das Ausschneidemittel (22) ein Strahlen emittierendes Gerät ist.

8. Atherektomiesystem nach irgendeinem der vorhergehenden Ansprüche, dadurch gekennzeichnet, daß ein proximaler Abschnitt des flexiblen rotierenden Katheters (15) einen gegenüber dem Durchmesser in seinem distalen Abschnitt verminderten Durchmesser (15') aufweist.

9. Atherektomiesystem nach irgendeinem der vorhergehenden Ansprüche, dadurch gekennzeichnet, daß der flexible Führungsdraht (17) sich radial erstreckende distale Sperrelemente (68) aufweist, um der distalen Bewegung von Obstruktionsmaterial entgegenzuwirken.

10. Atherektomiesystem nach Anspruch 9, dadurch gekennzeichnet, daß die distalen Sperrelemente (68) elastisch zusammenziehbar sind.

11. Atherektomiesystem nach Anspruch 9 oder 10, dadurch gekennzeichnet, daß die distalen Sperrelemente (68) selektiv ausdehnbar sind.

12. Atherektomiesystem nach irgendeinem der vorhergehenden Ansprüche, dadurch gekennzeichnet, daß Mittel (27) zum Eingeben von Fluiden in das Blutgefäß eines Patienten mit der Hülse (26) verbunden sind.

## Revendications

1. Système d'athérectomie pour l'extraction d'une obstruction hors du vaisseau d'un patient, comprenant :
un fil métallique de guidage souple (17) pouvant être introduit dans le vaisseau du patient ;
un cathéter rotatif souple (15) destiné à découper et à ingérer la matière créant l'obstruction, pouvant être introduit dans le vaisseau sur le fil de guidage souple, le cathéter rotatif souple (15) ayant, à son extrémité distale, des moyens de carottage rotatifs (22) en vue de pratiquer un découpage circulaire dans une obstruction située devant eux, et des moyens d'accouplement (30) à son extrémité proximale, en vue de le connecter à une source d'énergie (24), un passage continu défini entre le cathéter rotatif souple (15) et le fil de guidage souple (17), le cathéter rotatif souple (15) étant disposé rotatif et coulissant dans un manchon (26) avec une extrémité distale ouverte,
caractérisé en ce que
le manchon (26) comporte une fenêtre (26') au voisinage de ladite extrémité distale ouverte en vue de recevoir la matière d'obstruction, ledit cathéter étant agencé de manière à être déplaçable distalement au-delà de la fenêtre (26') pour découper et ingérer la matière d'obstruction dans le manchon (26).

2. Système d'athérectomie selon la revendication 1, caractérisé en ce que le manchon (26) présente une section transversale qui contraint positivement le cathéter souple (15) à demeurer à l'intérieur du manchon dans la région 35 de ladite fenêtre (26').

3. Système d'athérectomie selon la revendication 1 ou la revendication 2, caractérisé en ce que le manchon (26) comporte sur lui des moyens de décentrage (42) actionnables sélectivement pour amener la fenêtre (26') contre ladite obstruction.

4. Système d'athérectomie selon la revendication 3 caractérisé en ce que les moyens de décentrage consistent en une chambre gonflable (42) fixée au manchon (26) à l'opposé de la fenêtre (26').

5. Système d'athérectomie selon l'une quelconque des revendications précédentes, caractérisé par des moyens pour appliquer une aspiration à une extrémité proximale du cathéter rotatif souple (15) de manière à attirer proximalement la matière d'obstruction prélevée dans ledit cathéter.

6. Système d'athérectomie selon l'une quelconque des revendications précédentes, caractérisé en ce que les moyens rotatifs de carottage consistent en une lame tubulaire (22).

7. Système d'athérectomie selon l'une quelconque des revendications précédentes, caractérisé en ce que les moyens de carottage (22) consistent en un dispositif émetteur de radiations.

8. Système d'athérectomie selon l'une quelconque des revendications précédentes, caractérisé en ce qu'une portion proximale du cathéter rotatif souple (15) présente un diamètre réduit (15') par rapport à son diamètre dans la section distale.

9. Système d'athérectomie selon l'une quelconque des revendications précédentes, caractérisé en ce que le fil de guidage souple (17) présente des moyens d'arrêt distal s'étendant radialement (68) pour s'opposer au déplacement distal de la matière d'obstruction.

10. Système d'athérectomie selon la revendication 9, caractérisé en ce que les moyens d'arrêt distal (68) sont élastiquement contractables.

11. Système d'athérectomie selon la revendication 9 ou la revendication 10, caractérisé en ce que les moyens d'arrêt distal (68) sont sélectivement déployables.

12. Système d'athérectomie selon l'une quelconque des revendications précédentes, caractérisé en ce que des moyens (27) sont connectés au manchon (26) en vue d'introduire des fluides dans le vaisseau du patient.
